# EUROPEAN PATENT APPLICATION

(11) **EP 2 604 221 A2**
(43) Date of publication of application: **19.06.2013**
(21) Application number: 12195330.1
(22) Date of filing: 03.12.2012
(51) Int. Cl.: A61F 2/00

(54) **Fibrotic band interrupter and implant introducing device**

(30) Priority: 15.12.2011 US 201113326696
(71) Applicant: Insightra Medical, Inc., Irvine, California 92618 (US)
(72) Inventor: Noda, Wayne A., California, California 92692 (US); Adzich, Vaso, California, California 92705 (US); Bell, Stephen Graham, 00142 Roma (IT); Amato, Giuseppe, 90144 Palermo (IT)
(74) Representative: Schütte, Gearoid

(57) **Abstract**

A space in a muscle wall such as the inguinal canal is dilated by a plunger-based mechanism to break up fibrotic bands by divulsion. While the space is dilated a dynamic plug is advanced into it, with the plug expanding and contracting with the space.

## Description

### FIELD OF THE INVENTION

The present invention relates generally to the repair of defects in muscular structures, and more particularly to fibrotic band interrupter and implant introducing devices to advance implants into a patient for repairing direct and indirect inguinal hernias.

### BACKGROUND OF THE INVENTION

A hernia is a condition in which part of the intestine bulges through a weak area in muscles of the abdomen. An inguinal hernia, the most common of which are the "indirect" and the "direct" inguinal hernia, occurs in the groin area between the abdomen and thigh. In an inguinal hernia the intestines push through a weak spot in the inguinal canal, which is an opening between layers of abdominal muscle near the groin. Obesity, pregnancy, heavy lifting, and straining to pass stool can cause the intestine to push against the inguinal canal. As also recognized herein, other repeated minor trauma to the wall from, e.g., coughing and the like can create the weakness that develops into a hernia.

The main treatment for inguinal hernia is surgery to block the protrusion of abdominal content through the muscle wall. This surgery is called herniorrhaphy, and typically involves suturing the muscle layers and fascia together to reinforce the wall or blocking the defect with a flat polypropylene mesh, and in some procedures used with a static polypropylene plug which rests in the opening without exerting any pressure on the surrounding tissue. As understood herein, this procedure can lead to complications including pain, swelling, bleeding, as well as recurrence.

### SUMMARY OF THE INVENTION

According to the invention, there is provided an assembly comprising:
a housing including a hollow proximal barrel and a hollow distal bulb, the bulb and barrel being coaxial and being engaged with each other to define a continuous channel from a proximal end of the barrel to a distal opening of the bulb, the bulb having a larger outside diameter than the barrel and having a rounded distal segment tapering down to the distal opening, an interior stop being formed in the barrel and extending into the channel;
a closure element covering the proximal end of the barrel and defining a round opening and a keyway contiguous to the round opening; and
a plunger including a stem slidably disposed in the channel from the proximal end of the barrel into the channel and terminating at a distal disc, a proximal disc being formed on the stem, a key being formed on the stem, the plunger being movable relative to the housing between a distal position, in which the distal disc is closely juxtaposed with the distal opening of the bulb, an unlocked proximal position, in which the proximal disc abuts the interior stop to prevent proximal motion of the plunger relative to the housing and the key of the plunger aligns with the keyway of the closure element to permit distal motion of the plunger toward the distal position, and a locked proximal position, in which the plunger is at a different angular location relative to the barrel from an angular position of the plunger in the unlocked proximal position such that the key of the plunger is not aligned with the keyway of the closure element and instead the key abuts the closure element to prevent distal motion of the plunger relative to the housing.

In one embodiment of the invention, the channel has a first diameter in the barrel and a second diameter in the bulb, and the second diameter is larger than the first diameter.

In another embodiment the interior stop is an annular ring circumscribing the barrel.

In another embodiment, the barrel is cylindrical.

In another embodiment, the keyway includes a first rectilinear opening extending radially from the round opening of the closure element and a second rectilinear opening extending radially from the round opening of the closure element and radially opposed to the first rectilinear opening, and the key of the plunger includes radially opposed first and second ribs on the stem of the plunger slidably disposed in respective rectilinear openings of the keyway.

In another embodiment, the closure element includes radially opposed flat wings extending radially away from the barrel.

In a further embodiment, a stop flange may circumscribe a proximal end of the bulb, extending radially outwardly therefrom. The stop flange can define a circular periphery except for a notch formed in the periphery.

In another embodiment, an example implant for deployment using the apparatus can include a central core and plural resilient lamellae extending radially from the core to define an implant body. Preferably, the implant body is biased to an extend configuration, in which the lamellae establish a radially uniform configuration having an enlarged diameter, and is foldable to a deploy configuration, in which some of the lamellae are folded around the central core to establish a loop space on one side of the implant body and to configure the body to have a deploy diameter smaller than the enlarged diameter to facilitate advancing the implant into the bulb for deployment thereof.

In another embodiment, the notch is aligned with the loop space of the implant body when the implant is in the deploy configuration in the bulb to facilitate juxtaposing the loop space with a spermatic cord of a patient during deployment of the implant from the bulb.

In another aspect, the invention provides apparatus comprising:
a housing including a hollow proximal barrel and a hollow distal bulb, the distal bulb being curved from an open distal end such that a distal diameter of the bulb is smaller than a proximal diameter of the bulb so that the bulb can be advanced into an opening in tissue distal diameter first and then advanced further until the proximal diameter is in the opening so as to gently stretch the tissue during advancement of the bulb, thereby interrupting fibrotic bands in the tissue;
a plunger slidably disposed in the channel and terminating at a distal disc, the plunger being movable relative to the housing between a distal position, in which the distal disc is closely juxtaposed with the open end of the bulb, and a proximal position, in which the distal disc is distanced from the open end of the bulb such that a tissue repair implant is disposable in the open end of the bulb, wherein moving the plunger from the proximal position to the distal position urges the implant out of the bulb into tissue.

In another embodiment, the apparatus comprises:
an interior stop formed in the barrel and extending into a channel defined at least in part by the barrel;
a closure element covering a proximal end of the barrel and defining a round opening and a keyway contiguous to the round opening; and
wherein the plunger includes a stem slidably disposed in the channel from the proximal end of the barrel into the channel and terminating at the distal disc, a proximal disc being formed on the stem, a key being formed on the stem, the plunger being movable to an unlocked proximal position, in which the proximal disc abuts the interior stop to prevent proximal motion of the plunger relative to the housing and the key of the plunger aligns with the keyway of the closure element to permit distal motion of the plunger toward the distal position, the plunger being further movable to a locked proximal position, in which the plunger is at a different angular location relative to the barrel from an angular position of the plunger in the unlocked proximal position such that the key of the plunger is not aligned with the keyway of the closure element and instead the key abuts the closure element to prevent distal motion of the plunger relative to the housing.

In another embodiment, the channel has a first diameter in the barrel and a second diameter in the bulb, and the second diameter is larger than the first diameter.

In another embodiment, the interior stop is an annular ring circumscribing the barrel.

In another embodiment, the barrel is cylindrical.

In another embodiment, the keyway includes a first rectilinear opening extending radially from the round opening of the closure element and a second rectilinear opening extending radially from the round opening of the closure element and radially opposed to the first rectilinear opening, and the key of the plunger includes radially opposed first and second ribs on the stem of the plunger slidably disposed in respective rectilinear openings of the keyway.

In another embodiment the closure element includes radially opposed flat wings extending radially away from the barrel.

In another embodiment the apparatus further comprises a stop flange circumscribing a proximal end of the bulb and extending radially outwardly therefrom, the stop flange defining a circular periphery except for a notch formed in the periphery.

In another embodiment the apparatus further comprises an implant including a central core and plural resilient lamellae extending radially from the core to define an implant body, the implant body being biased to an extend configuration, in which the lamellae establish a radially uniform configuration having an enlarged diameter, and implant body being foldable to a deploy configuration, in which some of the lamellae are folded around the central core to establish a loop space on one side of the implant body and to configure the body to have a deploy diameter smaller than the enlarged diameter to facilitate advancing the implant into the bulb for deployment thereof.

In another embodiment the apparatus further comprises a stop flange circumscribing a proximal end of the bulb and extending radially outwardly therefrom, the stop flange defining a circular periphery except for a notch formed in the periphery, the notch being aligned with the loop space of the implant body when the implant is in the deploy configuration in the bulb to facilitate juxtaposing the loop space with a spermatic cord of a patient during deployment of the implant from the bulb.

In another aspect the invention provides a method comprising:
retracting a plunger in a hollow barrel and rotating the plunger to a locked proximal position such that the plunger cannot be advanced back distally into the barrel;
advancing an implant into an open distal end of a bulb extending proximally from the barrel;
advancing the bulb into a tissue defect until a depth stop flange formed between the bulb and barrel rests against a muscular wall in which the tissue defect is formed;
rendering the plunger into an unlocked proximal position;
advancing the plunger distally relative to the barrel to release the implant out of the open distal end onto the defect.

### BRIEF DESCRIPTION OF THE DRAWINGS

The details of the present invention, both as to its structure and operation, can best be understood in reference to the accompanying drawings, in which like reference numerals refer to like parts, and in which:
Figure 1 is a first perspective view of a fibrotic band interrupter and implant introducing device according to the invention, showing the plunger through the transparent barrel in the advanced position with an example implant schematically shown having been pushed out of the distal end;
Figure 2 is a second perspective view of the fibrotic band interrupter and implant introducing device, showing the plunger in the retracted position;
Figure 2A is a plan view of the proximal end of the barrel;
Figure 3 is a first side view of the fibrotic band interrupter and implant introducing device, showing the plunger in the advanced position;
Figure 4 is a second side view of the fibrotic band interrupter and implant introducing device, showing the plunger in the retracted position;
Figure 5 is a first end view of the fibrotic band interrupter and implant introducing device;
Figure 6 is a second end view of the fibrotic band interrupter and implant introducing device;
Figure 7 is a perspective view of an example implant having a mesh with integral plug portion woven into the mesh, with both the mesh and plug including strengthening members, showing two enlarged views to illustrate the strengthening members;
Figure 8 is a side view of an alternate interrupter with a movable stop flange in the retracted position, showing some structure in phantom; and
Figure 9 is a side view of the alternate interrupter shown in Figure 8 in the advanced position.

### DETAILED DESCRIPTION OF THE PREFERRED EMBODIMENT

As set forth in greater detail below, methods and devices are disclosed to dilate the muscular gap in which protrudes the hernia, as well as the inguinal canal, indeed in some embodiments the entire inguinal canal, for the purpose of rupturing fibrous bands within the muscle wall to allow a return to the natural physiological state of contraction/relaxation and, thus, blocking and at the same time accomplishing in dynamic fashion the action of the sphincter (or similar anatomical structures that function like a sphincter) even in the case of anterior or posterior open hernia procedure as well as during any appropriate type of laparoscopic approach such as TAPP or TEP. A dilating instrument may be used to dilate the opening for the above purpose. The dilating instrument may be engaged with a preloaded implant and if desired a force gauge mechanism (e.g. manometer) to precisely control the dilation forces.

A dynamic implant is deployed that contracts and relaxes with the inguinal canal. In addition, in non-limiting embodiments adhesions may be resolved to further release the muscle wall prior to dilation by, e.g., scrubbing the adhesions with a pad. When the delivery device is removed the newly restored contractibility of the muscle usually causes the muscle to grasp and hold the implant.

In addition to physical dilation of the defect, the fibrous bands can also be disrupted using mechanical, electrical, thermal, magnetic, or chemical processes. For example, direct or indirect stimulation by electricity of the muscles can be used to create deform spasms which through non-physiologic contraction break the fibrosis. Cooling or heating may be used at temperatures established to break the stiff connective fibres only within the muscles, thus breaking the fibrosis. Magnetic objects such as small magnetic or ferromagnetic discs or even small particles can be disposed in the inguinal ring through, e.g., a catheter and then a large magnet disposed external to the patient to attract the internal magnetic objects to stretch the muscles and cause divulsion. Enzymatic or chemical lysis of stiff connective fibres can be applied through, e.g., hyperdermic injection or perioperatively to break down only fibrotic tissue and not healthy tissue.

As described further below, the present implant preferably is a dynamic device that expands and contracts in a radial fashion in synchrony with the muscles of the entire inguinal canal to allow the contraction and relaxation of the sphincter in a normal physiological way. The implant is configured to exert a constant outward pressure on the surrounding tissue, and it may incorporate a unique lamellar structure at its core to allow the spermatic cord to slide freely along the length of the core, thus not impinging the cord structures and vessels. The implant may be made of materials that have low tissue reactivity. A solid, liquid gel or gas or combination of any to create the implant and can be made from autologous tissues, xenotissues or any form of biological material. The implant can be permanent, temporary, absorbable, non-absorbable or a combination of all of these and can be detected radiologically or by using other imaging diagnostic procedures. The implant can be, e.g., tubular, round, conical, irregular, regular etc. The implant may be retained within the defect by friction, i.e., by radial force between the implant and muscle wall. Also, the implant may sutured, clipped, or glued to the wall.

Application of the implant may use open abdomen surgery, laparoscopic surgery, endoscopic surgery, or a combination thereof. The implant can be deployed forwards or backwards, and the applicator can be temporary, permanent, non-absorbable, or absorbable.

The dynamic implant, device and/or the method for repairing and reinforcing tissue or muscular structures, according to the present invention can be used to repair all types of hernias including inguinal direct, indirect or mixed hernias, femoral, incisional, and recurrent hernias. The described method may be applicable to other similar muscular wall defects such as trans-septal cardiovascular defects. The scope of the methods of the invention are not restricted solely to hernia but include any body structure requiring divulsion of the surrounding muscular structures to restore contractility followed by the deployment of an implant with the intention to reinforce the muscular structure allowing a dynamic repair of the physiological functions of the structures. Other muscle walls with defects that may be treated using the systems and methods herein include the septum of the heart, anterior abdominal wall, diaphragm, female pelvic muscles such as levator ani, and the femoral canal.

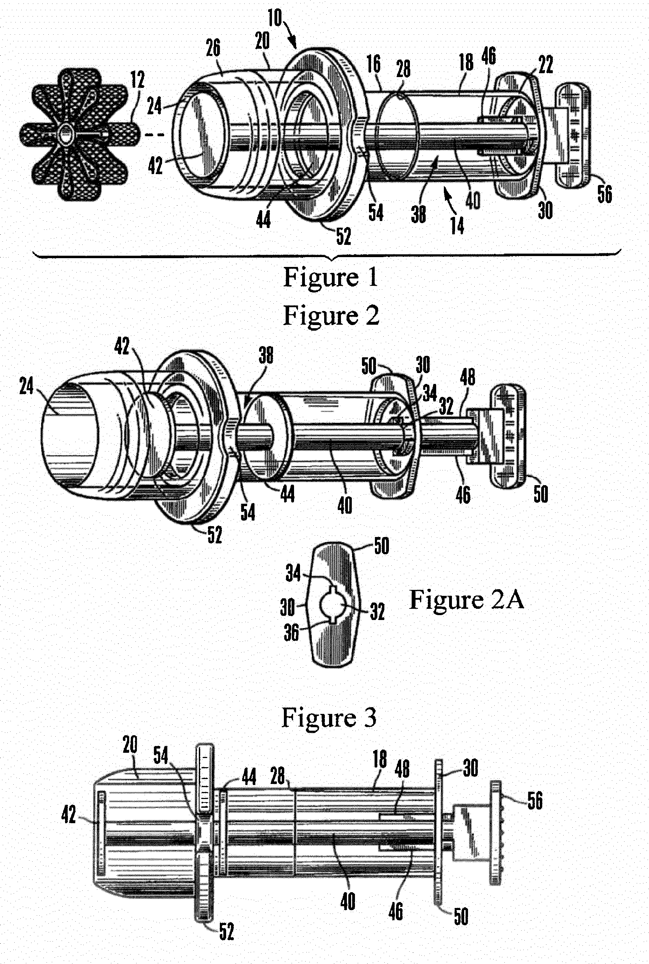

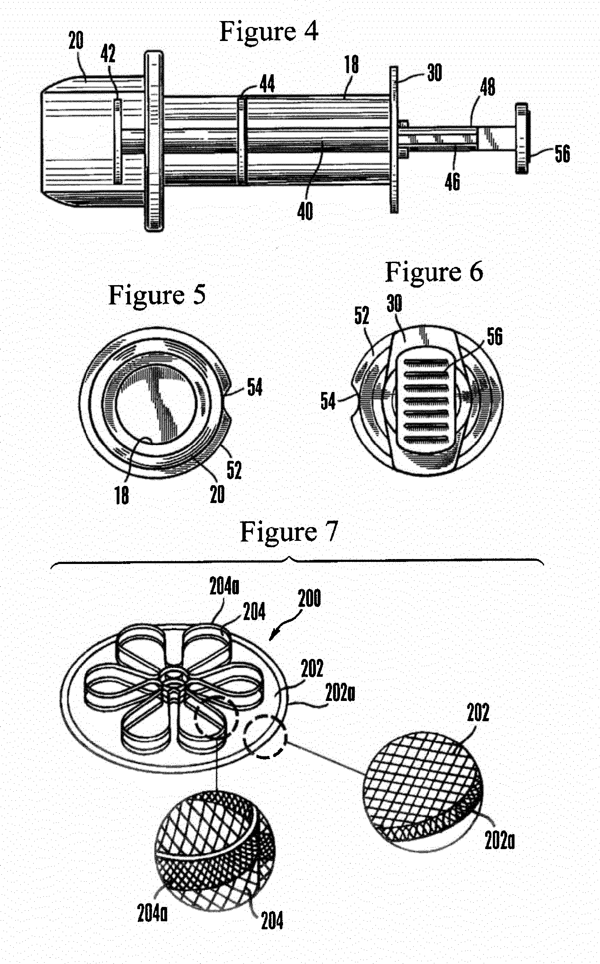

Referring to Figures 1-4, a system 10 includes an implant 12 that may be implemented by any of the implants discussed in the above-incorporated patent applications including the implant 200 shown in Figure 7 and discussed below. To hold the implant 12 and position it adjacent tissue to be repaired, at which point the implant is ejected onto the tissue, a fibrotic band interrupter and implant introducing device 14 is provided with a housing 16 that may be made of transparent or translucent plastic. The housing 16 includes a cylindrical hollow proximal barrel 18 and a hollow distal bulb 20, with the bulb 20 and barrel 18 being coaxial as shown and being engaged with each other to define a continuous channel from a proximal end 22 of the barrel 18 to a distal opening 24 of the bulb 20. The channel can have a first diameter in the barrel and a second diameter in the bulb, and the second diameter can be larger than the first diameter.

As also shown, the bulb 20 has a larger outside diameter than the barrel 18 and has a rounded distal segment 26 tapering down to the distal opening 24. Thus, the distal bulb 20 is curved from the open distal end 24 such that a distal diameter of the bulb is smaller than a proximal diameter of the bulb so that the bulb can be advanced into an opening in tissue distal diameter first and then advanced further until the proximal diameter is in the opening so as to gently stretch the tissue during advancement of the bulb, thereby interrupting fibrotic bands in the tissue. Stated differently, the diameter of the distal opening than the largest diameter of the bulb 20.

As best shown in Figure 3, an interior stop 28 is formed in the barrel 18 and extends into the channel formed by the barrel 18 and bulb 20. In the example shown, the interior stop is an annular ring circumscribing the barrel. Also, as best shown in Figures 2 and 2A, a closure element 30 covers the proximal end of the barrel 18 and defines a round opening 32 and an open keyway 34 contiguous to the round opening 32. In the example shown, the keyway includes a first rectilinear opening 34 extending radially from the round opening 32 of the closure element 30 and a second rectilinear opening 36 extending radially from the round opening 32 of the closure element 30 and radially opposed to the first rectilinear opening 34.

A plunger 38 including a stem 40 is slidably disposed in the channel from the proximal end of the barrel 18 into the channel, terminating at a distal disc 42. A proximal disc 44 is formed on the stem 40. Also, a key is formed on the stem 40. As best shown in cross-reference to Figures 2, 2A, and 3, the key of the plunger 38 in the example shown includes radially opposed first and second ribs 46, 48 on the stem 40 that are slideably disposed in respective rectilinear openings 34, 36 of the keyway.

With the above description in mind, the plunger 38 is movable relative to the housing between a distal position (Figures 1 and 3), in which the distal disc 42 is closely juxtaposed with the distal opening 24 of the bulb 20, and an unlocked proximal position (Figures 2 and 4), in which the proximal disc 44 rearwardly abuts the interior stop 28 to prevent proximal motion of the plunger 38 relative to the housing and in which the key 46, 48 of the plunger 38 aligns with the keyway 34, 36 of the closure element 30 to permit distal motion of the plunger 38 toward the distal position. Still further, the plunger can be rotated to a locked proximal position substantially in the same axial position as shown in Figures 2 and 4 but in which the plunger 38 is at a different angular location relative to the barrel from an angular position of the plunger 38 in the unlocked proximal position such that the key 46, 48 of the plunger 38 is not aligned with the keyway 34, 36 of the closure element 30. Instead, the key abuts the closure element to prevent distal motion of the plunger 38 relative to the housing.

In non-limiting examples, the closure element 30 may include radially opposed flat wings 50 extending radially away from the barrel. These serve the purpose of finger grips which enable single hand operation. Also, a stop flange 52 can circumscribe a proximal end of the bulb 20 and can extend radially outwardly therefrom. As shown, the stop flange 52 can define a circular periphery except for an alignment notch 54 (Figures 2, 3, and 5) formed in the periphery. As will become clearer in light of description below related to the preferred exmplae implant of Figure 7, when properly installed, the notch 54 is aligned with a loop space that is formed in the implant body when the implant is deformed into the deploy configuration in the bulb 20 to facilitate the visualization of juxtaposing the loop space with a spermatic cord of a patient during deployment of the implant from the bulb 20 by aligning the loop space with the notch 54 during installation. A thumb plate 56 (Figure 6) can be disposed on the proximal end of the plunger 38 as shown to receive a user's finger or thumb.

Figure 7 shows an implant device 200 that includes a round deformable thread mesh 202 designed to lay against a muscle wall and a ribbon or thread plug 204 engaged with the mesh 202 and designed to fill a hole in the muscle wall. The plug 204 is formed of a ribbon of mesh strands in a flower petal configuration as shown. The plug 204 alternatively may be a plug disclosed in the present assignee's U.S. patent application serial no. 11/934,897, incorporated herein by reference.

The plug 204 and/or mesh 202 may be provided with strengthening members in accordance with disclosure below. Briefly, in the non-limiting example shown strengthening members 202a may be provided around the periphery of the mesh 202 while strengthening members 204a are provided around the peripheries of the tops and bottoms of each "petal" of the plug 204.

In the example shown, the strengthening members 202a, 204a are established by thread fibres that are more closely knitted together than the fibres of the mesh 202/plug 204. The fibres of the strengthening members 202a, 204a, which individually may be the same size or smaller (e.g., a mil in diameter smaller) than the fibres of the mesh 202 and plug 204, are woven (including as by knitting or sewing) into the fibres of the mesh 202 and plug 204, respectively. This creates additional stiffness by concentrating more material in one area, resulting in increased fibre density, increased thickness, or both.

In example non-limiting embodiments the mesh 202 can be knitted in an open weave pattern, using polypropylene fibres three to eight mils in diameter. The mesh 202 can have a pore size of between eight-tenths of a square millimeter and sixteen square millimeters (0.8mm² - 16mm²). To establish the strengthening member 202a, polypropylene fibres of, e.g., three to eight mils in diameter are sewn around the edge of the mesh 202 in a close knit sewing pattern and/or multiple passes can be made around the edge to build up additional fibre density. For example, the fibre density of the strengthening member 202a may be ten to one hundred times the fibre density of the remainder of the mesh 202.

As an alternate means of construction stiffer regions with additional fibres, additional fibre material can be welded to the mesh material. Similarly to when the additional fibres are woven into the material, the additional fibres are integrated to the mesh material and cannot be easily removed.

For instance, a polypropylene mesh ring can be constructed of fibres three to eight mils in diameter. One to four additional rings, each one-tenth of an inch to a half an inch in width, of the same material and of the same outer diameter as the mesh 202 can then be welded onto the mesh 202. This creates additional fibre density (as viewed from the top) on the edge of the mesh 202, creating a stiffer material in selected locations, biasing the material in a wrinkle free condition.

Likewise, present principles set forth above contemplate that the plug 204 can have both stiffness and elasticity, so that the combination of structure has a resistance to crush, but can still return to an original configuration if deformed. In some embodiments the overall amount of material may be minimized, and the stiffness can be anisotropic. This may be achieved by increasing the fibre density in specific regions in the same manner as described above.

In greater detail, a knitted mesh material can be knitted into a strip with a more open knit in the middle (pore size of between eight-tenths of a square millimeter and sixteen square millimeters (0.8mm² - 16mm²) and significantly greater fibre density (length of fibre in a given area) at the edges (fibre density ten to one hundred times greater than in the base material) using a polypropylene fibre three to eight mils in thickness. This strip can then be heat set into a final weave configuration and further heat set into a petal configuration. This particular method creates resistance to circumferential crush on the sides of the petals, but minimal resistance to crush from the top.

As the fibre thickness increases, the stiffness increases, but the elasticity (i.e. the ability to return to a given shape after being deformed) decreases. Therefore, the amount of fibres and fibre thickness can be established to obtain the desired combination of stiffness and elasticity. Specifically, in example non-limiting embodiments the mesh 202/plug 204 can be knitted of a polypropylene fibre of between four to eight mils in diameter while the fibres that establish the strengthening members 202a, 204a can be one-half mil to three mils smaller in diameter than those used in the mesh 202/plug 204, and can be knitted to the edges of the mesh 202/plug 204 in a denser configuration to produce specific material properties. To increase the resistance to crush from the top, additional fibres may be knitted in a sinusoidal pattern into the middle of the plug 204.

In use, the plunger 38 is retracted relative to the barrel 18 and rotated, e.g., counterclockwise on the body to the locked proximal position. The loops of the implant 200 are then manually folded around the central core of the implant. In the folded position, the user inserts loop structures of the implant 200 into the distal end 24 of the bulb 20, aligning the space that is established between the loops by the folding action with the notch 54. This alignment, in the case of indirect inguinal hernia, helps the loops fold open around the spermatic cord when the implant subsequently is pushed out of the bulb 20.

Typically, the hernia or other tissue defect is prepared according to standard hernia technique. A preperitoneal space large enough to accommodate the implant disk can be created using finger guided blunt dissection of the peritoneal sheath from the abdominal wall. In any case, the bulb 20 with implant 200 located therein is advanced into the inguinal defect. As stated above, in the case of indirect inguinal hernia the notch 54 should be aligned with the spermatic cord to allow the implant 200 to unfold around the cord. If present, the cord should remain stretched laterally throughout the implantation procedure.

Note that the implant disk 202 remains distally outside the open end 24 of the bulb 20 as it is advanced. The bulb 20 is carefully advanced into the defect until the disk 202 of the implant 200 is fully behind the muscle wall in the preperitoneal space and the depth stop flange 52 rests against the external border of the muscular wall.

Once the disk 202 of the implant is in the preperitoneal space, the bulb 20 is gently retracted proximally toward the user until a resistance from the disk is felt. This signifies the implant is ready to be deployed. The plunger 38 is then rotated, e.g., clockwise until it stops, i.e., is in the unlocked proximal position shown in Figures 2 and 4. The barrel 18 with bulb 20 is carefully pulled proximally by the user while the angular position of the barrel relative to the abdominal plane is maintained. As the barrel 18 with bulb 20 is withdrawn, the user pushes the plunger 38 to the distal position shown in Figures 1 and 3 to release the implant 200, allowing the core of the implant to remain inside the defect. Following delivery of the implant, the core of the implant will be in the defect. The loops of the implant should be positioned evenly around the defect to form a complete circle. This can be done using surgical forceps. If a spermatic cord is present, care must be taken to ensure it lies between two of the loops. Using clinical judgment, a securing suture(s) may be placed between the loop(s) of the implant and the muscle wall. Monofilament, non-absorbable sutures may be used. Also, using clinical judgment an anterior mesh (not shown) may be added to help to reinforce the repair. The mesh should be secured to the core of the implant with one single monofilament suture.

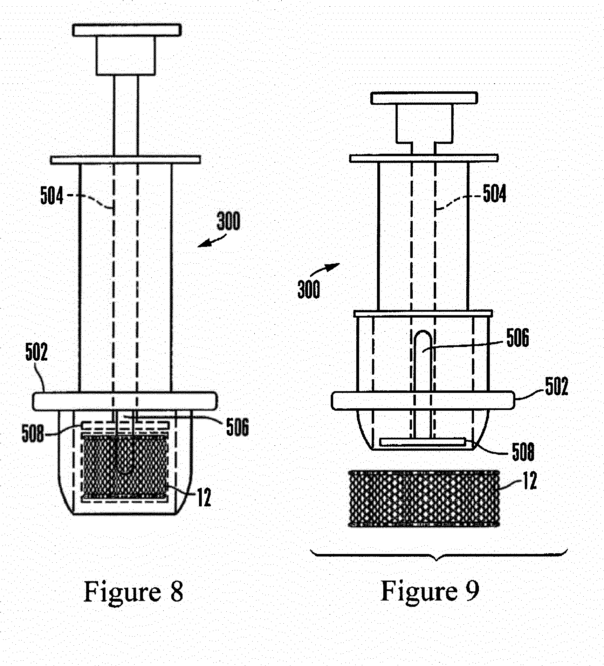

Figures 8 and 9 show an alternate interrupter 300 that is in all essential respects identical in construction and operation to the device 14 described above, except that the stop flange 502 is connected by a connector bar or segment (not shown) to the plunger 504 through a slot 506 such that the stop flange 502 moves with the plunger 504 (and thus with the distal disc 508). This insures that the implant 12 is deployed into the proper plane of the tissue defect and is not pushed distally beyond the proper plane into, e.g., the preperitoneal pocket, since the stop flange 502 will abut intervening tissue before excessive distal pushing occurs.

While the particular Fibrotic Band Interrupter and Implant Introducing Device is herein shown and described in detail, it is to be understood that the subject matter which is encompassed by the present invention is limited only by the claims.

## Claims

1. Assembly, comprising:
a housing including a hollow proximal barrel and a hollow distal bulb, the bulb and barrel being coaxial and being engaged with each other to define a continuous channel from a proximal end of the barrel to a distal opening of the bulb, the bulb having a larger outside diameter than the barrel and having a rounded distal segment tapering down to the distal opening, an interior stop being formed in the barrel and extending into the channel;
a closure element covering the proximal end of the barrel and defining a round opening and a keyway contiguous to the round opening; and
a plunger including a stem slidably disposed in the channel from the proximal end of the barrel into the channel and terminating at a distal disc, a proximal disc being formed on the stem, a key being formed on the stem, the plunger being movable relative to the housing between a distal position, in which the distal disc is closely juxtaposed with the distal opening of the bulb, an unlocked proximal position, in which the proximal disc abuts the interior stop to prevent proximal motion of the plunger relative to the housing and the key of the plunger aligns with the keyway of the closure element to permit distal motion of the plunger toward the distal position, and a locked proximal position, in which the plunger is at a different angular location relative to the barrel from an angular position of the plunger in the unlocked proximal position such that the key of the plunger is not aligned with the keyway of the closure element and instead the key abuts the closure element to prevent distal motion of the plunger relative to the housing.

2. The assembly as claimed in claim 1, wherein the channel has a first diameter in the barrel and a second diameter in the bulb, and the second diameter is larger than the first diameter.

3. The assembly as claimed in claim 1 or claim 2, wherein the interior stop is an annular ring circumscribing the barrel.

4. The assembly as claimed in any one of the preceding claims, wherein the barrel is cylindrical.

5. The assembly as claimed in any one of the preceding claims, wherein the keyway includes a first rectilinear opening extending radially from the round opening of the closure element and a second rectilinear opening extending radially from the round opening of the closure element and radially opposed to the first rectilinear opening, and the key of the plunger includes radially opposed first and second ribs on the stem of the plunger slidably disposed in respective rectilinear openings of the keyway.

6. The assembly as claimed in any one of the preceding claims, wherein the closure element includes radially opposed flat wings extending radially away from the barrel.

7. The assembly as claimed in any one of the preceding claims, further comprising a stop flange circumscribing a proximal end of the bulb and extending radially outwardly therefrom, the stop flange defining a circular periphery except for a notch formed in the periphery.

8. The assembly as claimed in any one of the preceding claims, further comprising an implant including a central core and plural resilient lamellae extending radially from the core to define an implant body, the implant body being biased to an extend configuration, in which the lamellae establish a radially uniform configuration having an enlarged diameter, the implant body being foldable to a deploy configuration, in which some of the lamellae are folded around the central core to establish a loop space on one side of the implant body and to configure the body to have a deploy diameter smaller than the enlarged diameter to facilitate advancing the implant into the bulb for deployment thereof.

9. The assembly of Claim 8, further comprising a stop flange circumscribing a proximal end of the bulb and extending radially outwardly therefrom, the stop flange defining a circular periphery except for a notch formed in the periphery, the notch being aligned with the loop space of the implant body when the implant is in the deploy configuration in the bulb to facilitate juxtaposing the loop space with a spermatic cord of a patient during deployment of the implant from the bulb.

10. Apparatus comprising:
a housing including a hollow proximal barrel and a hollow distal bulb, the distal bulb being curved from an open distal end such that a distal diameter of the bulb is smaller than a proximal diameter of the bulb so that the bulb can be advanced into an opening in tissue distal diameter first and then advanced further until the proximal diameter is in the opening so as to gently stretch the tissue during advancement of the bulb, thereby interrupting fibrotic bands in the tissue;
a plunger slidably disposed in the channel and terminating at a distal disc, the plunger being movable relative to the housing between a distal position, in which the distal disc is closely juxtaposed with the open end of the bulb, and a proximal position, in which the distal disc is distanced from the open end of the bulb such that a tissue repair implant is disposable in the open end of the bulb, wherein moving the plunger from the proximal position to the distal position urges the implant out of the bulb into tissue.

11. The apparatus as claimed in claim 10, comprising:
an interior stop formed in the barrel and extending into a channel defined at least in part by the barrel;
a closure element covering a proximal end of the barrel and defining a round opening and a keyway contiguous to the round opening; and
wherein the plunger includes a stem slidably disposed in the channel from the proximal end of the barrel into the channel and terminating at the distal disc, a proximal disc being formed on the stem, a key being formed on the stem, the plunger being movable to an unlocked proximal position, in which the proximal disc abuts the interior stop to prevent proximal motion of the plunger relative to the housing and the key of the plunger aligns with the keyway of the closure element to permit distal motion of the plunger toward the distal position, the plunger being further movable to a locked proximal position, in which the plunger is at a different angular location relative to the barrel from an angular position of the plunger in the unlocked proximal position such that the key of the plunger is not aligned with the keyway of the closure element and instead the key abuts the closure element to prevent distal motion of the plunger relative to the housing.

12. The apparatus as claimed in claim 10 or claim 11, wherein the channel has a first diameter in the barrel and a second diameter in the bulb, and the second diameter is larger than the first diameter.

13. The apparatus as claimed in any one of claims 10 to 12, wherein the interior stop is an annular ring circumscribing the barrel.

14. The apparatus as claimed in any one of claims 10 to 13, wherein the barrel is cylindrical.

15. The apparatus as claimed in any one of claims 10 to 14, wherein the keyway includes a first rectilinear opening extending radially from the round opening of the closure element and a second rectilinear opening extending radially from the round opening of the closure element and radially opposed to the first rectilinear opening, and the key of the plunger includes radially opposed first and second ribs on the stem of the plunger slidably disposed in respective rectilinear openings of the keyway.
